# EUROPEAN PATENT SPECIFICATION

(11) **EP 4 686 473 B1**
(45) Date of publication and mention of the grant of the patent: **28.10.2026**
(21) Application number: 25177063.2
(22) Date of filing: 16.05.2025
(51) Int. Cl.: A61K 31/194, A61K 31/565, A61K 31/77, A61K 9/00, A61K 9/70, A61K 47/10, A61K 47/34, A61P 35/00, A61P 35/04, A61K 47/12, A61K 9/08, A61K 9/127

(54) **PHARMACEUTICAL COMPOSITION, PHARMACEUTICAL COMPOSITION FORMULATION AND THEIR USE IN THE TREATMENT OF SKIN CANCER**
PHARMAZEUTISCHE ZUSAMMENSETZUNG, PHARMAZEUTISCHE ZUSAMMENSETZUNGSFORMULIERUNG UND DEREN VERWENDUNG BEI DER BEHANDLUNG VON HAUTKREBS
COMPOSITION PHARMACEUTIQUE, FORMULATION DE COMPOSITION PHARMACEUTIQUE ET LEUR UTILISATION DANS LE TRAITEMENT DU CANCER DE LA PEAU

(30) Priority: 30.07.2024 PL 44941324
(43) Date of publication of application: 04.02.2026
(73) Proprietor: Gdanski Uniwersytet Medyczny, 80-210 Gdansk (PL)
(72) Inventor: Gorska-Ponikowska, Magdalena, 80-442 Gdansk (PL); Izycka-Swieszewska, Ewa, 80-180 Gdansk (PL); Uzdrowska, Katarzyna, 80-180 Gdansk (PL); Baczek, Tomasz, 80-174 Gdansk (PL); Gulczynski, Jacek, 80-461 Gdansk (PL); Konieczna, Lucyna, 84-240 Reda (PL); Swierczewska, Zuzanna, 80-283 Gdansk (PL); Knap, Narcyz, 80-255 Gdansk (PL); Kostrzewa, Tomasz, 76-200 Slupsk (PL); Baranska-Rybak, Wioletta, 80-126 Gdansk (PL)
(74) Representative: Godlewski, Piotr

(56) References cited:
- WO-A1-2023/018342
- A. KAMM ET AL: "The cytotoxic properties of graphene derivatives and 2-metoxyestradiol / graphene compound on human melanoma (A375) and osteosarcoma (143B) cell lines", VIRCHOWS ARCHIV, vol. 481, no. S1, 16 August 2022 (2022-08-16), Berlin/Heidelberg, pages S340, XP093328339, ISSN: 0945-6317, DOI: 10.1007/s00428-022-03379-4

## Description

The object of the invention is a pharmaceutical composition, a preparation containing the pharmaceutical composition and the use of the pharmaceutical composition in the treatment of skin cancer. The invention is related to the field of medicine, particularly oncology, and has applications in oncological or palliative therapy.

Known from the Polish patent publication PL243445 B1 are GO-2-ME and rGO-2-ME hybrids constituting a stable combination of 2-methoxyestradiol (2-ME) molecules with graphene oxide GO or reduced graphene oxide rGO, the production method of these hybrids and their use as a therapeutic system of a relatively selective anti-cancer drug.

A composite material containing oxidised graphene containing the drug doxorubicin is known from the Chinese patent application CN104146946 A, where the oxidised graphene is modified with lactobionic acid.

Known from English patent application GB2532449 A are nanomaterials for use in the treatment, prevention or prevention of cancer by inhibiting the proliferation of cancer stem cells, where the nanomaterial is functionalised by at least one functional group containing a heteroatom selected from O, S and N.

There is no topical preparation on the pharmaceutical market with an anti-tumour, anti-inflammatory and regenerative effect on wounds such as cancer wounds. Therefore, patients with skin metastases, advanced skin and soft tissue tumours, patients in the terminal phase with cancerous ulcers who cannot be treated surgically, using radio nor chemotherapy, are only given symptomatic treatment. In the current state of the art, there is a very strong need to develop new and complex compositions and formulations with anti-tumour and anti-inflammatory properties, with high bioavailability, taking into account the ease of use and hygiene of local skin cancer conditions.

The aim of the present invention was to develop a pharmaceutical composition with an increased degree of local bioavailability of the active substance, with anti-cancer, anti-inflammatory and pro-regenerative effects, as well as reducing bacterial biofilm formation and with beneficial effects on damaged mammary cell membranes, and the use of this pharmaceutical composition in the treatment of primary and metastatic skin cancers, in particular advanced or terminal cancers. Another objective according to the invention was to develop a product and an application of the pharmaceutical composition which is easy to use and facilitates the maintenance of hygiene during the use of the pharmaceutical preparation, thus facilitating the course of treatment, patient care and improving the quality of life of oncology patients, in particular in the advanced or terminal stage of cancers with skin involvement.

According to the present invention, the Graph-2-ME formulation denotes a hybrid of graphene and 2-methoxyestradiol, wherein graphene is in the form of graphene oxide.

Surprisingly, all the aforementioned technical problems have been solved by the present invention.

The object of the invention is a pharmaceutical composition, characterised in that it contains between 5.0 and 15.0 weight% of poloxamer 188, no more than 5.0 weight% of polidocanol, no more than 1.0 weight% of diethylene glycol monoethyl ether, no more than 10.0 weight% of glycerine, no more than 2.0 weight% of phenoxyethanol and no more than 5.0 weight% of graphene oxide modified with 2-methoxyestradiol.

The composition according to the invention preferably also contains no more than 1.0 weight% of a pH regulator.

The pH regulator is preferably lactic acid or sodium or potassium lactate.

Another object of the invention is a preparation containing the pharmaceutical composition according to the invention.

The preparation according to the invention preferably has a physical form for topical application allowing direct contact with the affected part of the skin.

The physical form of the preparation is preferably selected from a group including: aerosol, spray, ointment, cream or gel.

The preparation according to the invention is preferably a film-forming preparation.

Another subject of the invention is the use of the pharmaceutical composition or preparation according to the invention in the treatment of skin and soft tissue tumours, fistulas, ulcers, particularly of cancerous origin or tumours associated with skin metastases.

The tumour is preferably selected from a group including: melanoma, squamous cell carcinoma, osteosarcoma, ulcer-associated tumours.

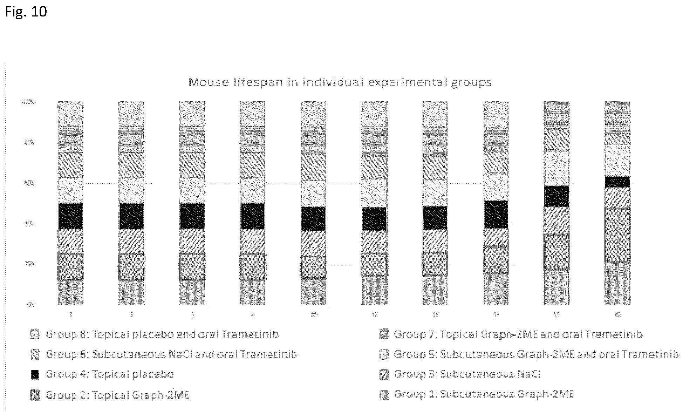

The subject of the invention in its embodiments is illustrated in the figure, in which Fig. 1 shows a schematic division of mice into experimental groups in a toxicity study of a pharmaceutical composition containing Graph-2-ME (procedure 1A and 1B), Fig. 2 depicts a schematic division of mice into experimental groups for testing the therapeutic potential of a pharmaceutical composition containing Graph-2-ME (procedures 2 and 3), Fig. 3 shows a schematic division of mice into experimental groups in a study of the therapeutic potential of the pharmaceutical composition containing Graph-2-ME (procedure 4 and 5), Fig. 4 shows a representative viability analysis, Fig. 5 shows a representative cell number analysis, Fig. 6 shows a graph illustrating the distribution of tumour growth rates in the groups administered the topical drug with Graph-2-ME and placebo, Fig. 7 depicts a graph showing the distribution of tumour growth rates in the course of a combination therapy (combination therapy II), as well as monotherapy with the topical drug, where the combination therapy includes the administration of the topical drug containing the active substance Graph-2-ME and the oral administration of a drug (Trametinib), while monotherapy includes only the administration of the topical drug containing the active substance Graph-2-ME, Fig. 8 shows a graph presenting the distribution of tumour growth rates in the course of combination therapy including administration of a topical drug containing the active substance Graph-2-ME and oral drug administration (Trametinib), as well as monotherapy comprising only administration of the topical drug containing the active substance Graph-2-ME, Fig. 9 shows pictures of the tumours in the mice under study in the last (third) week of therapy, wherein pictures 8a, 8b, 8c illustrate tumours in the group with topical administration of the drug containing the active substance Graph-2-ME, and pictures 8d, 8e, 8f illustrate tumours in the group using placebo, Fig. 10 shows a graph depicting the lifespan of the mice in the different study groups.

The advantage according to the invention is the reduction of local and general symptoms such as pain, itching, exidate from the wound and accelerated healing of ulcers and fistulas. Another advantage is that the composition according to the invention can be used against any conditions involving the skin. An important advantage is that the composition according to the invention can be used in any form, as an aerosol/spray, while maintaining the topical administration regime.

### Example 1 - Pharmaceutical compositions

The pharmaceutical compositions in the form of an aerosol preparation have been developed such that, in addition to the anti-tumour effect, they also have anti-inflammatory, anti-pruritic, anaesthetic and protective (protective) effects against ulcerative wounds in the course of advanced skin cancers. The overall qualitative and quantitative composition of the pharmaceutical compositions is shown in Table 1, while the most preferable qualitative and quantitative composition is shown in Table 2. All ingredients in the compositions are pharmacopoeial ingredients that are approved for use in medicinal products, the so-called pharmaceutically acceptable ingredients.

**Table 1. Overall quantitative and qualitative composition of pharmaceutical compositions according to the invention.**

| No. | Pharmacopoeial component | Weight content [%] | Function |
|---|---|---|---|
| 1 | Poloxamer 188 | 5.00 - 15.00 | Active substance, thermo-gelling agent |
| 2 | Polidocanol (macrogol lauryl ether) | ≤ 5.00 | Active substance |
| 3 | Diethylene glycol monoethyl ether | ≤ 10.00 | Active substance |
| 4 | Glycerine | ≤ 10.00 | Active substance |
| 5 | Phenoxyethanol | ≤ 2.00 | Preservative |
| 6 | Graphene oxide modified with 2-methoxyestradiol (Graph-2-ME) | ≤ 5.0 | Active substance |
| 7 | Sodium lactate/lactic acid | ≤ 1.0 | pH regulator (quantity depends on the need) |
| 8 | Water | ≥ 52.00 | Dispersing phase (quantity depends on the need) |

**Tab. 2. Quantitative and qualitative composition of the most preferable pharmaceutical composition according to the invention.**

| No. | Pharmacopoeial component | Weight content [%] | Function |
|---|---|---|---|
| 1 | Poloxamer 188 | 13.00 | Active substance, thermo-gelling agent |
| 2 | Polidocanol (macrogol lauryl ether) | 3.00 | Active substance |
| 3 | Diethylene glycol monoethyl ether | 5.00 | Active substance |
| 4 | Glycerine | 5.00 | Active substance |
| 5 | Phenoxyethanol | 1.10 | Preservative |
| 6 | Graph-2-ME | 3.21 | Active substance |
| 7 | Sodium lactate/lactic acid | Q. S. | PH adjuster |
| 8 | Water | 69.69 | Dispersing phase |

### Example 2 - Method of manufacturing a pharmaceutical composition

The present method of manufacturing a pharmaceutical composition covers only an exemplary production method and does not limit the subject matter of the invention to this example only.

The method of manufacturing a pharmaceutical composition requires the use of a balance with a high degree of accuracy. First, 52.0 g of sterile demineralised water is weighed into the vessel as the dispersing phase. The water is then heated to 50-55°C. Once the desired temperature is reached, 15.0 g of poloxamer 188 is added and stirred at min. 600 rpm, until the solution is completely homogenised. The mixture is cooled to room temperature, i.e. in the range of 15-25°C, while stirring during the cooling is continued. In the next step, 5.0 g of polidocanol is added and mixed until a homogeneous solution is obtained. Next the following ingredients are added successively: 10.0 g of diethylene glycol monoethyl ether, 10.0 g of glycerol, 2.0 g of phenoxyethanol and 5.0 g of Graph-2-ME are added, after which this prepared mixture system is stirred for at least 10 minutes, until a homogeneous solution is obtained. Finally, the pH of the mixture is verified and the pH is stabilised, preferably using sodium lactate (as a base) or lactic acid (as an acid) to set the pH at the physiological skin pH of 5.5 ± 0.5. Any other pharmaceutically acceptable regulator known in the state of the art can be used as a pH regulator.

### Example 3 - Experimental studies

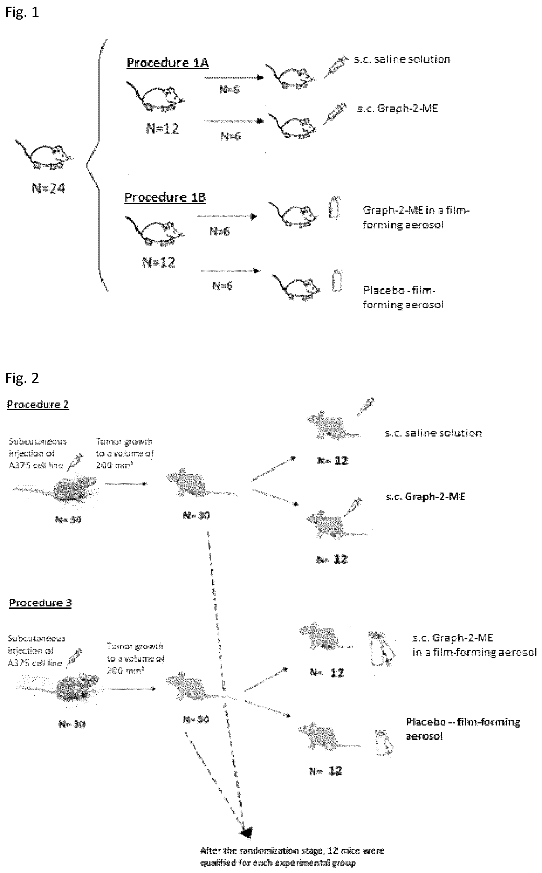

The experimental studies consists of two stages: the first stage of the study was carried out on 24 BALB/c female mice obtained from home breeding (TAZD) to assess the toxicity of the Graph-2ME compound in the developed composition. The general course of the first stage of the experiment, together with the division into groups, is shown in Fig. 1. It is important that no spontaneous death was observed in the test individuals during the toxicity assessment test of the Graph-2ME compound in the developed composition. All mice in each of the four groups studied during the course of the first stage of the study lived to the last (twenty-first) day of the first stage of the study. The subcutaneous administration was carried out on the left side of the body during this study stage. On the other hand, topical administration took place on a shaved patch of formerly hairy mouse skin, also in the left side area of the body. A 0.4 mm (27G) needle was used for subcutaneous administration in BALB/c mice in the first stage of the study. In subcutaneous administrations of a drug containing the active substance Graph-2ME, the section of skin to which the subcutaneous drug was applied was washed daily before the administration of next dose of the subcutaneous drug. Analogous procedures were followed for the subcutaneous administration of placebo.

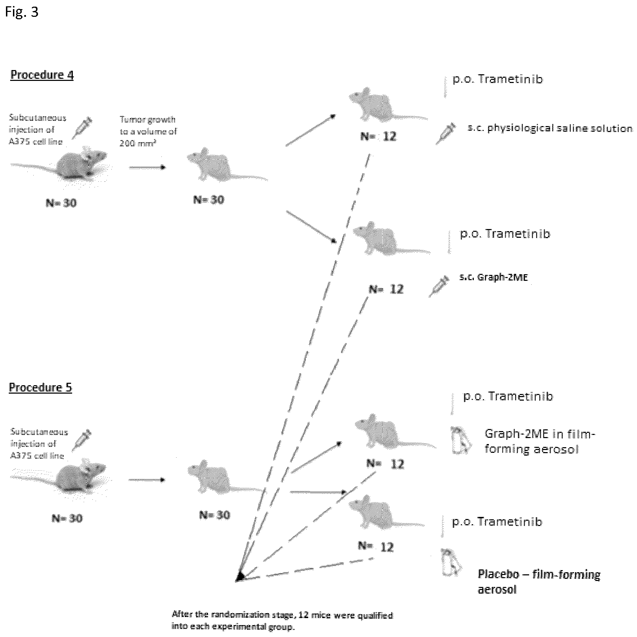

The aim of the second stage of the study was to assess the therapeutic potential of the Graph-2ME compound in subcutaneous application, and as an active substance in a topical drug. This study was performed on 120 Crl:CD-1-Foxn1'nu female mice purchased from a licensed supplier [Charles River, Germany]. The study was conducted both as monotherapy - procedures 2 and 3 (Fig. 2), and as combination therapy together with an orally administered drug (trametinib at a dose of 1 mg/kg body weight) - procedures 4 and 5 (Fig. 3). Upon arrival at the centre, the animals were quarantined for fourteen days and then familiarised with the person conducting the experiment (activity 1). Prior to administration in the respective study groups, tumour cells were implanted subcutaneously into the animals to produce xenograft tumours of malignant melanoma of the skin. Cells of the A725 line were used for this purpose.

In order to specify the therapeutic indications and form of anticancer therapy, the effect of an aerosol containing the active substance Graph-2-ME and the effect of a subcutaneous injection of the active substance Graph-2-ME alone were compared in the course of this experimental study.

The developed pharmaceutical formulation was a film-forming aerosol containing the active substance GO-2ME together with a carrier system. The subcutaneous injection, on the other hand, was a suspension of the Graph-2ME compound in physiological saline solution.

A comparison of the effects of the membrane-forming aerosol and the subcutaneous injection was carried out to observe the efficacy of therapy administered in both transdermal (membrane-forming aerosol) and injected applications. The results of the comparative analysis allow precise specification of the therapeutic indications and route of administration of the preparation containing the active substance Graph-2ME.

The aerosol preparation developed by the team may find use in oncological and palliative therapy in patients with advanced, inoperable tumours, in diffuse disease with skin metastases or fistulas, in the terminal phase of cancer or supportively during standard therapies. For this reason, the experiment envisaged testing the efficacy of a combination therapy of an aerosol with the active ingredient Graph-2ME and an injectable suspension of the Graph-2ME compound together with the anticancer drug Trametinib (procedure 4 and procedure 5) used in the treatment of melanoma. Trametinib is a drug which is a MEK inhibitor with anticancer activity (it inhibits MEK1 and MEK2).

In the course of the experiment, tumour volume was measured during random mice assignment to the different study groups. Mice with tumour growth to a minimum volume of 200 mm³ were included in the study groups. On the first day of treatment (after the randomisation process), no tumour ulceration was observed in any of the qualified mice. Tumour volume measurements, as well as skin analysis using a dermatoscope, were carried out during the experiment.

All topical and subcutaneous administrations in the second stage of the study were performed in the left side area of the body of the mice. In subcutaneous administrations of a drug containing the active substance Graph-2ME (group 2 and group 7), the section of skin to which the subcutaneous drug was applied was washed daily before the administration of next dose of the subcutaneous drug. An analogous procedure was followed for the subcutaneous administration of placebo (in study groups 4 and 8). Subcutaneous injections in the second stage of the study using CrI:CD-1-Foxn1'nu mice (applicable to groups: 1, 3, 5, 6) were performed over the tumour, using a 0.6 mm 23G needle. The change in needle diameter in the first (0.4 mm 27G) and second (0.6 mm 23G) stages of the study was due to the fact that, for practical reasons, the administration of the active substance Graph-2ME in saline solution forming a suspension of higher viscosity using a 0.4 mm diameter needle was extremely difficult with the co-existing ulcers and tumours.

The general course of the second stage of the experiment including the division into groups is shown in Figs. 2 and 3.

After 21 days of therapy in the study, the mice were terminated by bleeding from the heart and tissues (skin, brain, liver, kidneys, lungs) were collected from the mice for further analysis.

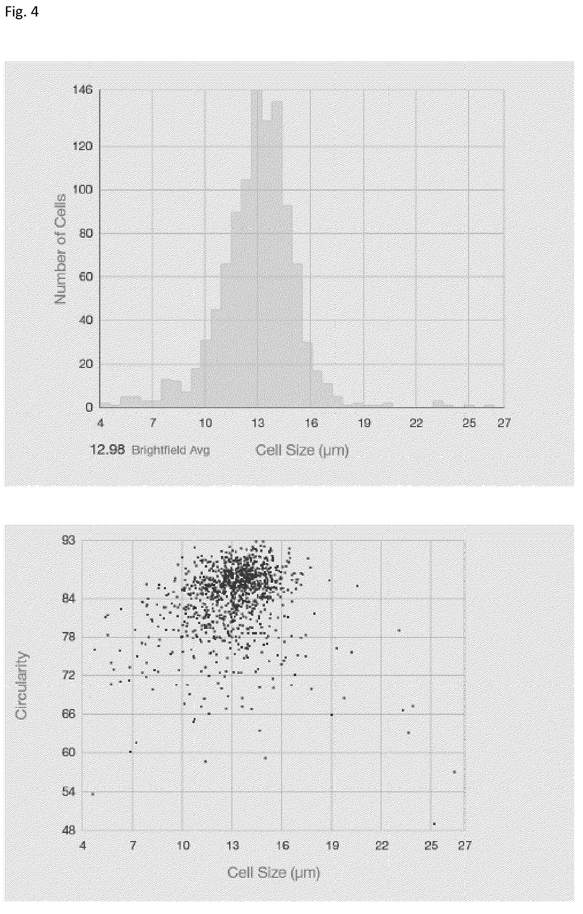

The A375 cell line (ATTC: CRL-1619), which was used to develop xenograft tumours in the studied mice, was cultured according to the protocol in a DMEM medium (Sigma, D6429) containing 15% FBS serum (Sigma, F7524) and 1% penicillin/streptomycin (Sigma, F4333). The culture was maintained under standard conditions of 37°C ambient temperature, 5%CO₂ and 95% humidity. The cell cultures were maintained until 80% confluence was reached, then trypsinised and transferred to further 175 cm² culture bottles until quantities sufficient for xenograft transplantation were obtained. Some of the cells were frozen in case the culture had to be reconstituted. Cells were assessed for mycoplasma infection twice, after thawing and during the culture growth, by DAPI staining and detection using fluorescence microscopy, which yielded negative results on each occasion. Cell viability during the culture growth period, as assessed by fluorescence staining with the ReadyCount^{™} Green/Red Viability Stain kit (ThermoFisher: A49905) and detection with the Countess III FL counter (ThermoFisher), was at 98-99%. On the day of implantation of the A375 cell line (passage 12) into the Crl:CD-1-Foxn1'nu mice, 8 ampoules containing 3.0x107 cells/mL each were prepared in a matrigel solution (MERCK, CLS354234) : HBBS (Sigma, H8264) (1:3, v/v). Cells from two culture vessels were counted before suspension in the mixture and viability was analysed using fluorescent staining and the ReadyCount^{™} Green/Red Viability Stain kit (ThermoFisher: A49905) (Fig. 4), while the Countess III FL (ThermoFisher) counter was used in detection (Tab. 2). The cells were characterised by a viability in the range of 98-99%. Cells were implanted into mice subcutaneously, as a 100 µL does of suspension.

**Table 3. Viability analysis of A375 cells by fluorescence staining.**

| Analysis | Number of cells | Cell viability |
|---|---|---|
| 1 | 5.55x10⁶/mL | 99% |
| 2 | 3.88x10⁶/mL | 98% |
| 3 | 5.26x10⁶/mL | 99% |
| 4 | 5.30x10⁶/mL | 99% |
| 5 | 4.31x10⁶/mL | 98% |
| 6 | 5.07x10⁶/mL | 98% |
| 7 | 4.48x10⁶/mL | 98% |
| 8 | 4.09x10⁶/mL | 99% |

Animals in which the tumour was not obtained after subcutaneous administration of A375 tumour cells or its size was insufficient for the subsequent steps planned in the individual procedures to be carried out, were put under anaesthesia through the use of isoflurane and then terminated by cervical vertebrae dislocation.

For the purpose of statistical evaluation, it was assumed that at the end of the experiment the tumour size in the untreated group would be approximately 1500 mm³. From the data identified in the literature, it could be concluded that intra-group variability can be as high as 25%. Using the prediction for the Student's t-test (comparison of treated and untreated group), assuming a test strength of 0.8 and p<0.5, the group size was calculated as 12 animals. However, due to the risk of transplant failure (lack of tumour growth), which according to the literature is approximately 25%, this number was increased to 15 animals. Twelve mice were randomised to each of the different study groups according to the predictions, after randomisation.

A list of all experimental groups participating in the study is shown in Table 3. The drug was administered daily for 21 days in each group.

**Tab. 4 List of experimental groups.**

| Group no. | Experimental group | Procedure no. | Number of animals per group |
|---|---|---|---|
| **1** | **Study group** - evaluation of the toxicity of the Graph-2-ME drug administered subcutaneously. Administration of Graph-2ME at a dose of 4.5 mg in 45-50 µl of saline solution (1:10 ratio). | Procedure 1A | 6 |
| **2** | **Study group** - evaluation of the toxicity of the Graph-2-ME drug administered topically, as aerosol. The dose of the active substance Graph-2-ME in a single subcutaneous administration is 4.5 mg. | Procedure 1B | 6 |
| **3** | **Control group for group 1 -** evaluation of the toxicity of the Graph-2-ME carrier (saline solution without drug - 0.9% NaCl) administered subcutaneously. Subcutaneous administration of of 45-50 µL. | Procedure 1A | 6 |
| **4** | **Control group for group 2** - evaluation of the toxicity of the Graph-2-ME drug carrier (a film-forming aerosol without the therapeutic substance) administered subcutaneously. Placebo group. | Procedure 1B | 6 |
| **5** | **Study group** - antitumour effect of the administered drug (2ME-Graph) as subcutaneous injection in the immediate vicinity of the tumour. Administration of Graph-2ME at a dose of 4.5 mg in 45-50 µL of saline solution (1:10 ratio). | Procedure 2 | 12 |
| **6** | **Control group for group 5** (a physiological saline solution will be administered instead of the drug - graphene). Subcutaneous administration of 45-50 µL of 0.9% NaCl. | Procedure 2 | 12 |
| **7** | **Study group** - antitumour effect of graphene administered topically to the tumour area in a film-forming spray. The dose of the active substance Graph-2-ME in a single subcutaneous administration is 4.5 mg. | Procedure 3 | 12 |
| **8** | **Control group for group 7 -** administration of graphene-free film-forming spray only. Placebo group. | Procedure 3 | 12 |
| **9** | **Study group** - antitumour effect of graphene administered by subcutaneous injection into the tumour area in combination therapy with orally administered trametinib. Administration of Graph-2ME at a dose of 4.5 mg in 45-50 µL of saline solution (1:10 ratio). Trametinib administered orally at a dose of 1 mg/kg body weight in 0.2 mL. | Procedure 4 | 12 |
| **10** | **Control group for group 9-** antitumour effect of physiological saline solution administered as subcutaneous injection into the tumour area in combined therapy with orally administered trametinib. Subcutaneous administration of 45-50 µL of 0.9% NaCl. Trametinib administered orally at a dose of 1 mg/kg body weight in 0.2 mL. | Procedure 4 | 12 |
| **11** | **Study group** - anti-cancer effect of graphene administered topically as a film-forming spray to the tumour area in combined therapy with tramenitib administered orally. The dose of the active substance Graph-2-ME in a single subcutaneous administration is 4.5 mg. Trametinib administered orally at a dose of 1 mg/kg body weight in 0.2 mL. | Procedure 5 | 12 |
| **12** | **Control group for group 11** - anti-cancer effect of film-forming aerosol (placebo) administered topically to the tumour area in combined therapy with tramenitib administered orally. Trametinib administered orally at a dose of 1 mg/kg body weight in 0.2 mL. | Procedure 5 | 12 |

### Example 4 - Results of experimental studies

The results of the measurements of the tumour volume formed from the A375 tumour cells implanted into the animals are shown in Table 4. The study demonstrates that the topically applied anticancer drug inhibited tumour growth during the study by 56.8% ± 14.02% (study groups: 2 and 4). In these studies, group 2 (topical drug with Graph-2ME) was tested against a placebo administered topically (group 4).

The active substance in subcutaneous administration resulted in a tumour growth inhibition by 10.4 ± 6.73% during the therapy (study groups 1 and 3). In this study, group 1 (Graph-2ME administered subcutaneously) was tested against saline solution (NaCl 0.9%) administered subcutaneously (group 3). This means that the active substance Graph-2ME administered subcutaneously has lower anti-tumour efficacy than a drug containing the same dose of the active substance Graph-2ME in topical administration. However, it should be taken into account that the topical administration took place daily and the frequency of subcutaneous administration of the active substance (and subcutaneous administration of saline as a control group for subcutaneous administration of the active substance Graph-2ME) took place twice a week. The subcutaneous administration of the active substance twice a week is caused by the fact that Graph-2ME suspension in saline requires a 0.6 mm (23G) needle. With this type of injection needle, and with the welfare of the mice in mind, it was necessary to limit the frequency of subcutaneous Graph-2ME administrations to twice a week (Mondays and Thursdays).

In the case of combination therapy I - a study of subcutaneous administration of the Graph 2-ME compound together with the oral drug Trametinib (groups 5 and 6), no inhibitory effect on tumour growth was observed.

The results of combination therapy II (topically administered drug) together with the oral drug Trametinib (study group 7) indicate tumour inhibition of 57.4 ± 9.77% during the study. This result is slightly higher than in study groups 2 and 4, where the topical drug was used as monotherapy. The tumour growth inhibition result was calculated relative to group 4 (topical placebo), due to the fact that control group 8 (topical placebo and Trametinib oral) did not survive to the end of the study.

**Table 5. Tumour volume measurement results on the first and last day of the study in each group.**

| Group | | Start d12 T1 (04/03/24) | d33_T22 (25/03/24) | |
|---|---|---|---|---|
| | | Tumour volume V [mm³] | Tumour growth V [mm³] | Tumour growth inhibition [%] |
| Group 1 | Graph-2ME subcutaneously | 286.0 | 2,691.1 | 10,4 ± 6,73 |
| Group 2 | Graph-2ME topically | 270.9 | 1,142.6 | 56,8 ± 14,02 |
| Group 3 | NaCl subcutaneously | 277.2 | 3,003.3 | - |
| Group 4 | Placebo topically | 272.8 | 2,645.4 | - |
| Group 5 | Graph-2ME subcutaneously and Trametinib orally | 285.1 | 3,236.4 | No tumour growth inhibition was observed |
| Group 6 | NaCl subcutaneously and Trametinib orally | 279,8 | 2,761.0 | - |
| Group 7 | Graph-2ME topically and Trametinib orally | 266.6 | 1,010.3 | 57.4 ± 977 |
| Group 8 | Placebo topically and Trametinib orally | 279.3 | The humanitarian termination occurred in this group on day 19 of the study. | |

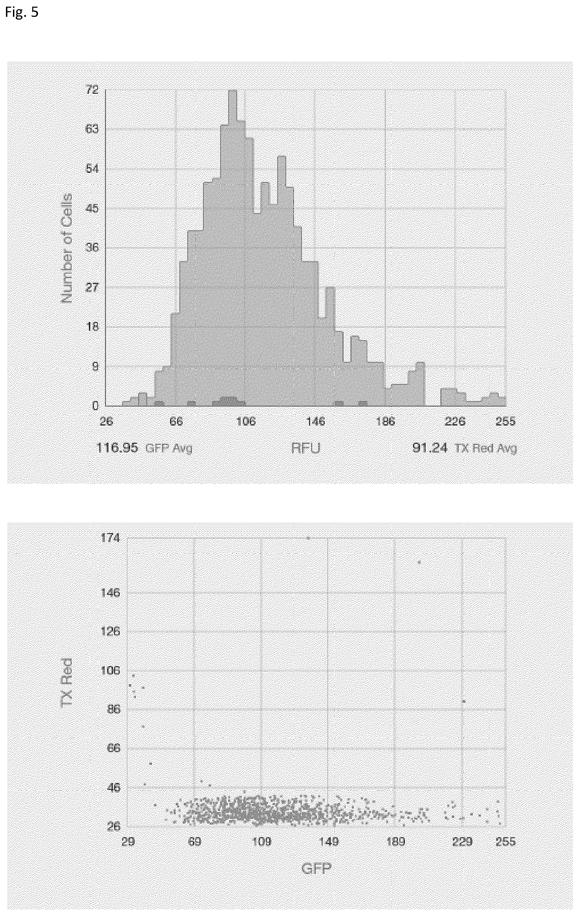

The results of the efficacy tests of the pharmaceutical composition are presented graphically as the ratio of the tumour volume on day n of the measurement (Vn) to the initial tumour size on the first day of the study (V0). Thus, the value of Vn / V0 on the first day of the test is always 1. The results clearly prove (Fig. 5) that the pharmaceutical composition according to the invention, when administered topically, inhibits the growth of xenograft tumours formed from A375 line cells. The graph also shows that the inhibitory effect of topical administration of the pharmaceutical composition on tumour growth occurs as early as the fifth day of the therapy.

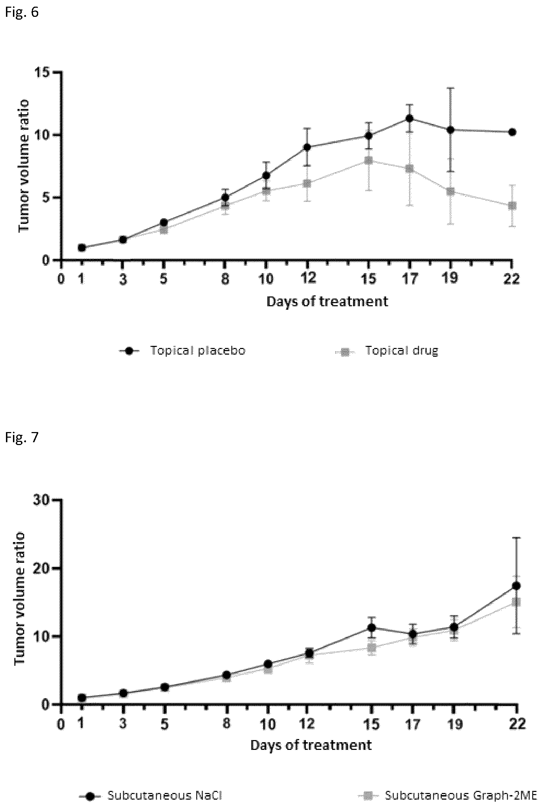

The results of other studies show that the pharmaceutical composition according to the invention, when administered subcutaneously, inhibits the growth of xenograft tumours formed from A375 line cells to a much less dynamic degree (much weaker) than the composition administered topically. It is inferred from the results graph (Fig. 6) that the tumour growth inhibitory effect of subcutaneous administration of the active substance Graph-2ME appears on the eighth day of the therapy. Thus, the first results of the therapy take three days longer to manifest than in the case of treatment with a topical drug.

Combination therapy (Fig. 7) involves the topical administration of a pharmaceutical composition with the active ingredient Graph-2ME and the oral administration of the drug (Trametinib), whereas monotherapy involves only the topical administration of a pharmaceutical composition containing the active ingredient Graph-2ME. The results were compared against placebo. The graph in Fig. 6 proves that the combination therapy resulted in a slightly more dynamic reduction in the volume of the xenograft tumour produced from A375 line cells than in the case of the monotherapy, where only the topical drug was used. It can also be seen that the tumour growth inhibitory effect of both the combination therapy comprising topical administration of the composition containing the Graph-2ME active substance and oral administration of the drug (Trametinib) and monotherapy including only topical administration of Graph-2ME occur similarly quickly - on day five of the therapy.

### Example 5 - Dermatoscopic evaluation of mouse skin in the different study groups

Dermatoscopic examination is a non-invasive diagnostic method used to assess skin lesions using a special device - a dermatoscope. Thanks to its magnification and built-in image illumination, the dermatoscope enables morphological assessment of the skin. Items such as the lesion colour, its borders, asymmetries or the presence of additional structures are assessed. In the described study, cancerous tumours were assessed - their shape, colour, visibility of blood vessels and the presence of pathological structures.

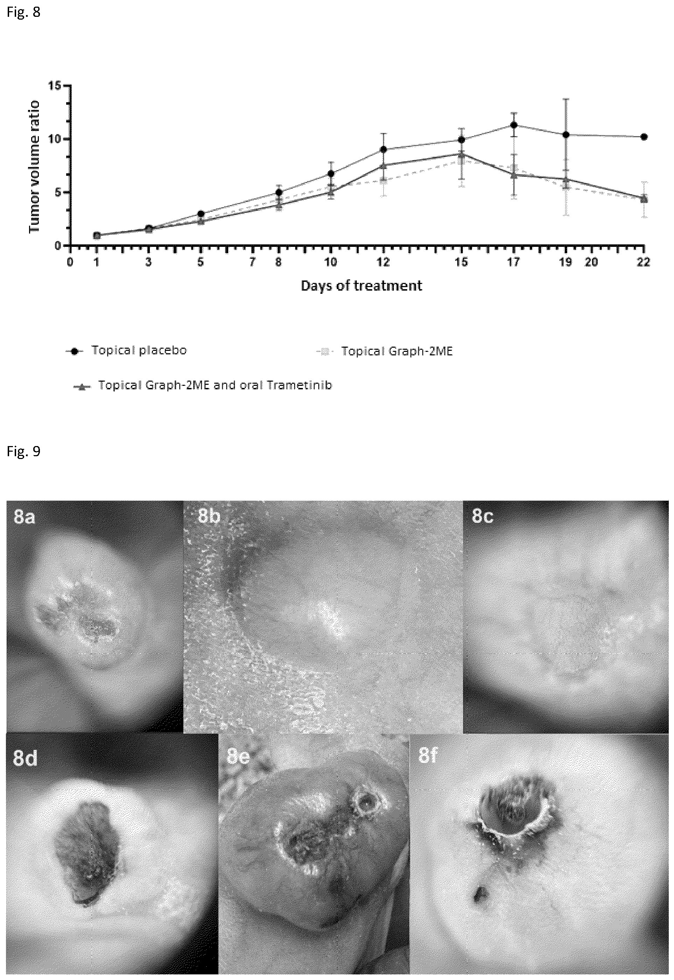

Dermatoscopic assessment of the skin of the test mice at the tumour growth site was performed weekly during the treatment. The results of this evaluation are shown in Table 5, and images of the tumours of the study mice from the last week of treatment for the group with topical administration of the composition containing the active ingredient Graph-2ME and the group with placebo administration are summarised in Fig. 8. During the last week of the study, crater-like ulcerations up to 0.5 cm in diameter were observed in the tumour area in the group with topical administration of the drug. At the same time, crater-like ulcerations up to 2.5 cm in diameter were preserved in the tumour area in mice from the topical placebo group. This means that the drug administered topically inhibited ulcer formation in the tumour area by up to five times better in the last week of therapy.

**Table 6. Dermatoscopic assessment of the skin of the study mice during the following weeks of treatment.**

| **Week 1** | | |
|---|---|---|
| Group 1 | Graph-2ME, subcutaneously | The skin over the assessed tumour was pink during the dermatoscopic assessment. No signs of irritation or eczema were present. Locally visible forming ulcerations approximately 1 mm in diameter were the result of natural tumour growth. |
| Group 2 | Graph-2ME topically | The skin over the assessed tumour was pink. No signs of irritation or eczema were present. Locally visible forming ulcerations approximately 1-2 mm in diameter were the result of natural tumour growth. |
| Group 3 | NaCl subcutaneously | The skin over the assessed tumour was pink. No signs of irritation or eczema were present. Widened vascular pattern visible in spots. |
| Group 4 | Placebo, topically | The skin over the assessed tumour was pink. No signs of irritation or eczema were present. Locally visible forming ulcerations approximately 1-1.5 mm in diameter were the result of natural tumour growth. |
| Group 5 | Graph-2ME subcutaneously and Trametinib orally | The skin over the assessed tumour was pink in colour, without pathological lesions. No signs of irritation or eczema were present. |
| Group 6 | NaCl subcutaneously and Trametinib orally | The skin over the assessed tumour was pink. No signs of irritation or eczema were present. Locally visible forming ulcerations approximately 1 mm in diameter were the result of natural tumour growth. |
| Group 7 | Graph-2ME topically and Trametinib orally | The skin over the assessed tumour was pink. No signs of irritation or eczema were present. Widened vascular pattern visible in places. |
| Group 8 | Placebo topically and Trametinib orally | The skin over the assessed tumour was pink. No signs of irritation or eczema were present. Widened vascular pattern visible in places. |

| **Week 2** | | |
|---|---|---|
| Group 1 | Graph-2ME, subcutaneously | The skin over the assessed tumour was pink. No signs of irritation or eczema were present. There are visible ulcerations 1 mm to 1 cm in diameter around the tumour. Subcutaneously administered preparation (graphene deposits?) visible in places. |
| Group 2 | Graph-2ME topically | The skin over the assessed tumour was pink. No signs of irritation or eczema were present. In the area of the tumour, ulcerations of approximately 1-4 mm in diameter were visible. Widened vascular pattern visible in places. |
| Group 3 | NaCl subcutaneously | The skin over the assessed tumour was pink. No signs of irritation or eczema were present. In the area of the tumour, ulcerations of approximately 1-10 mm in diameter were visible. Widened vascular pattern visible in places. |
| Group 4 | Placebo, topically | The skin over the assessed tumour was pink. No signs of irritation or eczema were present. In the area of the tumour, ulcerations of approximately 1-4 mm in diameter were visible. Widened vascular pattern visible in places. |
| Group 5 | Graph-2ME subcutaneously and Trametinib orally | The skin over the assessed tumour was pink. No signs of irritation or eczema were present. In the area of the tumour, ulcerations of approximately 1-5 mm in diameter were visible. Widened vascular pattern visible in places. |
| Group 6 | NaCl subcutaneously and Trametinib orally | The skin over the assessed tumour was pink. No signs of irritation or eczema were present. In the area of the tumour, ulcerations of approximately 1-3 mm in diameter. Widened vascular pattern visible in places. |
| Group 7 | Graph-2ME topically and Trametinib orally | The skin over the assessed tumour was pink. No signs of irritation or eczema were present. In the area of the tumour, ulcerations of approximately 1-5 mm in diameter. Widened vascular pattern visible in places. |
| Group 8 | Placebo topically and Trametinib orally | The skin over the assessed tumour was pink. No signs of irritation or eczema were present. There are visible ulcerations 1-3 mm in diameter around the tumour. |

| **Week 3** | | |
|---|---|---|
| Group 1 | Graph-2ME, subcutaneously | The skin over the assessed tumour was pink. No signs of irritation or eczema were present. Crater-like ulcerations up to 2 cm in diameter were present around the tumour. |
| Group 2 | Graph-2ME topically | The skin over the assessed tumour was pink. No signs of irritation or eczema were present. Crater-like ulcerations up to 0,5 cm in diameter were present around the tumour. |
| Group 3 | NaCl subcutaneously | The skin over the assessed tumour was pink. No signs of irritation or eczema were present. Crater-like ulcerations up to 2,5 cm in diameter were present around the tumour. |
| Group 4 | Placebo, topically | The skin over the assessed tumour was pink. No signs of irritation or eczema were present. Crater-like, bleeding ulcerations up to 2,5 cm in diameter were present around the tumour. |
| Group 5 | Graph-2ME subcutaneously and Trametinib orally | The skin over the assessed tumour was pink. No signs of irritation or eczema were present. In the area of the tumour, ulcerations of approximately 1-7 mm in diameter were visible. Visible widened vascular pattern persists. |
| Group 6 | NaCl subcutaneously and Trametinib orally | The skin over the assessed tumour was pink. No signs of irritation or eczema were present. In the area of the tumour, ulcerations of approximately 1-5 mm in diameter were visible. Visible widened vascular pattern persists. |
| Group 7 | Graph-2ME topically and Trametinib orally | The skin over the assessed tumour was pink. No signs of irritation or eczema were present. In the area of the tumour, ulcerations covered with scabs and up to 1 cm in diameter were visible. Visible widened vascular pattern persists. |
| Group 8 | Placebo topically and Trametinib orally | The skin over the assessed tumour was pink. No signs of irritation or eczema were present. In the area of the tumour, ulcerations covered with scabs 1,5 cm approximately in diameter were visible. |

### Example 6 - Life expectancy results for mice in the different study groups

The number of mice in each study group over the course of the study was determined by the use of a humanitarian termination. The symptoms underlying the decision to terminate the mice early are provided in Table 7.

**Table 7. Symptoms underlying the decision to terminate the test animals early.**

| **Type of lesion** | **Symptoms** |
|---|---|
| Individual and appearance changes | Lethargy of animals, excessive aggression, tousled coat, sensitivity to touch, chronic diarrhoea, lack of self-hygiene |
| Neurological changes | Paresis of limbs, coordination disorders, blindness, convulsions |
| Changes in feeding | Lack of food and/or water intake |
| Changes in body weight | Weight loss of more than 20% |
| Tumour volume | Above 2000 mm³ |

Due to the use of humanitarian termination in the course of therapy, mice from each study group were euthanised when humanitarian termination was necessary. Thus, the number of mice in each treatment group changed frequently on successive treatment days. A summary of the number of mice in each treatment group is shown in Table 8.

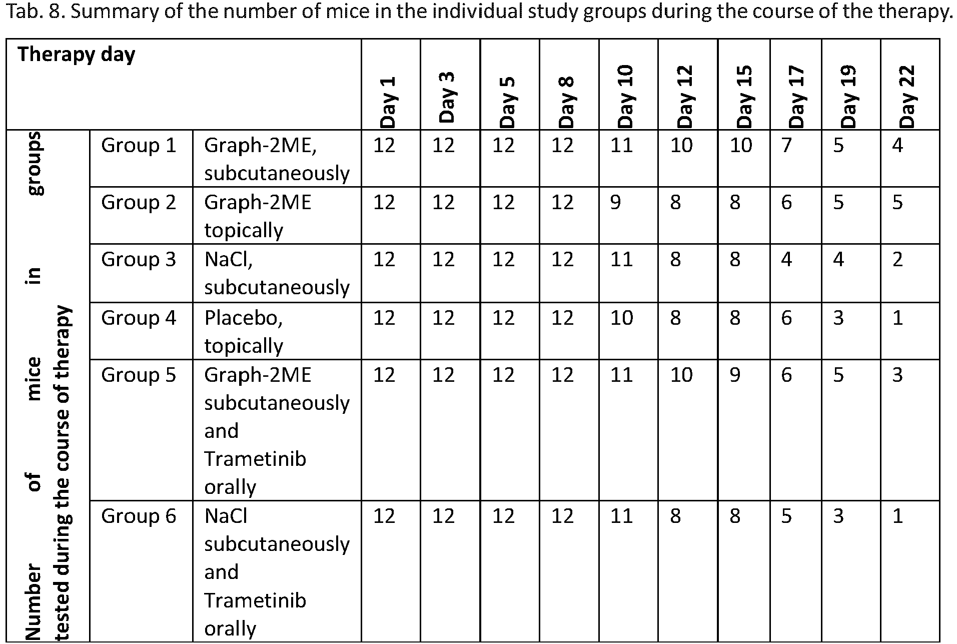

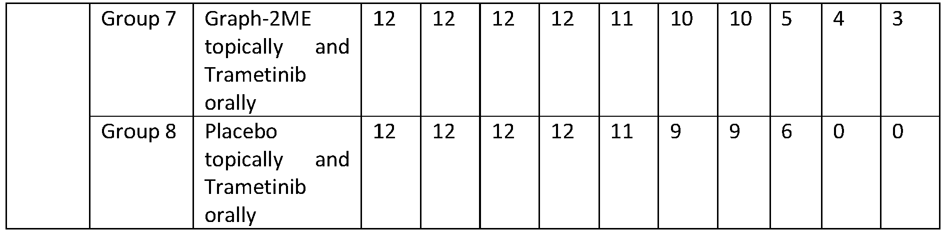

The lifespan of the mice in the individual treatment groups during the course of therapy is shown in Fig. 9. It can be inferred from the graph that the lifespan of the mice in the individual groups changed on the successive days of treatment. It is noteworthy that on the last day of treatment (day 22), group 2, i.e. the group of mice with topical administration of the anti-tumour composition according to the invention, was the group with highest viability. Group 2 had both the highest percentage of live mice on the last day of the study (26.32%).

The lifespan of the mice in the individual study groups was determined on the basis of the data from the last, i.e. the 22nd day of the study. 19 mice had survived by the last day of the study. A summary of the percentage of live mice in each study group and the percentage of mice surviving therapy within the groups are shown in Table 9. Group 2 not only had the highest percentage of live mice on the last day of the study (26.32%), but also the highest percentage of therapy survival among the mice enrolled in the group (41.67%). Trametinib was used as a positive control - as a modern drug used in the treatment of melanoma - but its efficacy compared to the composition according to the invention was very low.

**Table 9: Summary of the percentage of live mice in each study group and the percentage of mice which survived the treatment within the groups.**

| **Study group** | | **Number of live mice on the last day of the study** | **Percentage of live mice on the last day of the study [%]** | **Percentage of mice which survived therapy in the group [%]** |
|---|---|---|---|---|
| Group 1 | Graph-2ME, subcutaneously | 4 | 21.05 | 33.33 |
| Group 2 | Graph-2ME topically | 5 | 26.32 | 41.67 |
| Group 3 | NaCl, subcutaneously | 2 | 10.53 | 16.67 |
| Group 4 | Placebo, topically | 1 | 5.26 | 8.33 |
| Group 5 | Graph-2ME subcutaneously and Trametinib orally | 3 | 15.79 | 25.00 |
| Group 6 | NaCl subcutaneously and Trametinib orally | 1 | 5.26 | 8.33 |
| Group 7 | Graph-2ME topically and Trametinib orally | 3 | 15.79 | 25.00 |
| Group 8 | Placebo topically and Trametinib orally | 0 | 0.00 | 0.00 |
| **Total** | | **19 mice** | **100%** | |

### Final conclusions:

In addition to anti-cancer activity, the pharmaceutical composition according to the invention administered locally and topically, preferably in the form of a film-forming aerosol, has the task of stimulating regenerative processes and anti-inflammatory activity against the present skin tumours, ulcers. Previous in vitro studies of the used therapeutic substance Graph-2ME on healthy skin and cancer cell models have shown its selective cytotoxicity towards cancer cells only. The hybrid of graphene (in the form of graphene oxide) with the cytostatic 2-ME (2-methoxyestradiol) enhances the therapeutic effect of 2-ME, as graphene oxide (GO) has a positive effect on the healing process in the treatment of chronic wounds. In vivo and in vitro studies demonstrate that graphene oxide (GO-2-ME) promotes wound healing through migration and proliferation of fibroblasts and keratinocytes and regulates angiogenesis.

## Claims

1. A pharmaceutical composition, **characterised in that** it contains between 5.0 and 15.0 weight% of poloxamer 188, no more than 5.0 weight% of polidocanol, no more than 1.0 weight% of diethylene glycol monoethyl ether, no more than 10.0 weight% of glycerine, no more than 2.0 weight% of phenoxyethanol and no more than 5.0 weight% of graphene oxide modified with 2-methoxyestradiol.

2. Composition according to Claim 1, **characterised in that** it additionally contains no more than 1.0 weight% of the pH regulator.

3. Composition according to Claim 1, **characterised in that** the pH regulator is lactic acid or sodium or potassium lactate.

4. A preparation containing the pharmaceutical composition according to any of the Claims 1-3.

5. Preparation according to Claim 4, **characterised in that** it has the physical form appropriate for topical use, enabling direct contact with the part of the skin with pathological lesions.

6. Preparation according to any of the Claims 4-5, **characterised in that** the physical form of the preparation is selected from a group including: aerosol, spray, ointment, cream or gel.

7. A preparation according to any of the Claims 4-6, **characterised in that** it is a film-forming preparation.

8. A pharmaceutical composition defined according to any of the Claims 1-3 or a preparation defined according to any of the Claims 4-7 for use in the treatment of skin and soft tissue tumours, fistulas, ulcers, particularly of cancerous origin or tumours associated with skin metastases.

9. A pharmaceutical composition for use according to Claim 8, **characterised in that** the tumour is selected from a group including: melanoma, squamous cell carcinoma, osteosarcoma, ulcer-associated tumours.

## Patentansprüche

1. Eine pharmazeutische Zusammensetzung, **dadurch gekennzeichnet, dass** sie zwischen 5,0 und 15,0 Gew.-% Poloxamer 188, höchstens 5,0 Gew.-% Polidocanol, höchstens 1,0 Gew.-% Diethylenglykolmonoethylether, höchstens 10,0 Gew.-% Glycerin, höchstens 2,0 Gew.-% Phenoxyethanol und höchstens 5,0 Gew.-% mit 2-Methoxyestradiol modifiziertes Graphenoxid enthält.

2. Eine Zusammensetzung nach Anspruch 1, **dadurch gekennzeichnet, dass** sie zusätzlich höchstens 1,0 Gew.-% eines pH-Regulators enthält.

3. Eine Zusammensetzung nach Anspruch 1, **dadurch gekennzeichnet, dass** der pH-Regulator Milchsäure oder Natrium- oder Kaliumlactat ist.

4. Eine Zubereitung, die pharmazeutische Zusammensetzung nach einem der vorhergehenden Ansprüche 1 bis 3 enthält.

5. Eine Zubereitung nach Anspruch 4, **dadurch gekennzeichnet, dass** sie eine für die äußere Anwendung geeignete physikalische Darreichungsform aufweist, die einen direkten Kontakt mit dem Hautbereich ermöglicht, der von pathologischen Läsionen betroffen ist.

6. Eine Zubereitung nach einem der vorhergehenden Ansprüche 4 bis 5, **dadurch gekennzeichnet, dass** die Darreichungsform der Zubereitung aus einer Gruppe ausgewählt ist, die Aerosol, Spray, Salbe, Creme oder Gel umfasst.

7. Eine Zubereitung nach einem der vorhergehenden Ansprüche 4 bis 6, **dadurch gekennzeichnet, dass** es sich um eine filmbildende Zubereitung handelt.

8. Eine pharmazeutische Zusammensetzung nach einem der vorhergehenden Ansprüche 1 bis 3 oder eine Zubereitung nach einem der vorhergehenden Ansprüche 4 bis 7 zur Anwendung bei Behandlung von Haut- und Weichteilgewebetumoren, Fisteln und Geschwüren, insbesondere maligner Herkunft, oder von Tumoren, die mit Hautmetastasen einhergehen.

9. Eine pharmazeutische Zusammensetzung zur Anwendung nach Anspruch 8, **dadurch gekennzeichnet, dass** der Tumor aus einer Gruppe ausgewählt ist, die Melanom, Plattenepithelkarzinom, Osteosarkom, mit Geschwüren assoziierte Tumoren umfasst.

## Revendications

1. Une composition pharmaceutique, **caractérisée en ce qu'**elle contient entre 5,0 et 15,0 % en poids de poloxamère 188, au plus 5,0 % en poids de polidocanol, au plus 1,0 % en poids d'éther monoéthylique de diéthylène glycol, au plus 10,0 % en poids de glycérine, au plus 2,0 % en poids de phénoxyéthanol et au plus 5,0 % en poids d'oxyde de graphène modifié par du 2-méthoxyestradiol.

2. Composition selon la revendication 1, **caractérisée en ce qu'**elle contient en outre au plus 1,0 % en poids d'un régulateur de pH.

3. Composition selon la revendication 1, **caractérisée en ce que** le régulateur de pH est l'acide lactique ou le lactate de sodium ou de potassium.

4. Préparation contenant la composition pharmaceutique selon l'une quelconque des revendications 1 à 3.

5. Préparation selon la revendication 4, **caractérisée en ce qu'**elle présente une forme physique appropriée à une utilisation topique, permettant un contact direct avec la partie de la peau présentant des lésions pathologiques.

6. Préparation selon l'une quelconque des revendications 4 à 5, **caractérisée en ce que** la forme physique de la préparation est choisie dans le groupe comprenant : un aérosol, un spray, une pommade, une crème ou un gel.

7. Préparation selon l'une quelconque des revendications 4 à 6, **caractérisée en ce qu'**elle est une préparation filmogène.

8. Composition pharmaceutique telle que définie selon l'une quelconque des revendications 1 à 3 ou préparation telle que définie selon l'une quelconque des revendications 4 à 7, pour utilisation dans le traitement des tumeurs de la peau et des tissus mous, des fistules, des ulcères, notamment d'origine cancéreuse, ou des tumeurs associées à des métastases cutanées.

9. Composition pharmaceutique pour utilisation selon la revendication 8, **caractérisée en ce que** la tumeur est choisie dans le groupe comprenant : le mélanome, le carcinome épidermoïde, l'ostéosarcome et les tumeurs associées à des ulcères.
